# EUROPEAN PATENT SPECIFICATION

(11) **EP 1 297 783 B1**
(45) Date of publication and mention of the grant of the patent: **19.01.2005**
(21) Application number: 01121541.5
(22) Date of filing: 10.09.2001
(51) Int. Cl.: A61B 5/0205

(54) **A blood pressure monitor and a method for determining a risk rate for a disease**
Blutdrucküberwachung und Verfahren zur Bestimmung des Krankheitsrisikos
Appareil de surveillance de la préssion sanguine et procédé pour determiner le risque de maladie

(43) Date of publication of application: 02.04.2003
(73) Proprietor: Microlife Intellectual Property GmbH, 9442 Berneck (CH)
(72) Inventor: Lin, Kin Yuan, Taipei, 114 (TW)
(74) Representative: Hepp, Dieter

(56) References cited:
- EP-A- 0 590 200
- EP-A- 0 627 190
- US-A- 5 638 823
- PATENT ABSTRACTS OF JAPAN vol. 1999, no. 05, 31 May 1999 (1999-05-31) & JP 11 047096 A (OMRON CORP), 23 February 1999 (1999-02-23)

## Description

The invention relates to a blood pressure monitor and to a method for measuring the blood pressure and for determining a risk rate for a disease according to the preamble of the independent patent claims.

In recent years, there have been a progressive decrease in cardiovascular mortality in North America, Western Europe, Japan and Australasia. At the same time, the control of hypertension in these regions has considerably improved. The risk of suffering from cardiovascular diseases, however, does not only depend on the blood pressure levels of a patient but also on a plurality of other risk factors, which may or may not be applicable to the individual patient.

Known blood pressure monitors allow the user to measure the blood pressure. The risk of suffering from cardiovascular disease is determined by the doctor on the basis of the blood pressure values communicated to him and in consideration of the risk factors applicable to the patient.

From JP11-047096A (or patent abstracts of Japan Vol. 1999, no. 05), there is known a health control system which allows to enter data such as age, sex, weight, blood pressures and the like. This system is used to calculate a risk. All data have to be entered manually.

EP-627 190A is directed to a method of measuring the degree of arteriosclerosis. By means of a blood pressure monitor, a pressure/volume curve for a specific person is determined. The device allows the user to enter specific values such as the age of the person.

US-5,638,823A is directed to a method for detecting a coronary artery disease. Clinical data are automatically combined with acoustic signals representing heart sounds of a patient.

Up to now, there are no known devices which automatically help to indicate to the user a specific risk of suffering from a disease during blood pressure measurements. It is an object of the present invention to overcome the drawbacks of the prior art.

The blood pressure monitor according to the invention shall be easy to manufacture and designed for an easy use. It is a further object of the invention to provide a blood pressure monitor which can be used to determine the risk of suffering from cardiovascular diseases by a plurality of users, e.g. the members of a family.

It is still a further object of the invention to provide a system, which allows an objective stratification of such risks.

It is still a further object of the invention to provide a method for measuring the blood pressure of a patient and for determining a risk rate for suffering from a disease such as a cardiovascular disease for the specific patient.

According to the invention, these and other objects are solved with a blood pressure monitor and with a method according to the characterising portion of the independent patent claims.

The blood pressure monitor is on the one hand designed for measuring the blood pressure of a patient and for determining a risk rate for the patient of suffering from a disease on the other hand. In context with the present invention, the main focus are cardiovascular diseases. The invention may, however, be used for risk stratification of other types of diseases.

The blood pressure monitor comprises a measuring unit for measuring at least one blood pressure value. The measuring unit is formed as a conventional blood pressure monitor.

The blood pressure monitor additionally includes a user input interface, which allows to enter data of at least one predetermined risk factor, which is relevant for the risk of suffering from said disease. The blood pressure monitor is further provided with a memory for storing these data. The memory is coupled to the interface. The blood pressure monitor is also provided with a calculating unit coupled to the memory and coupled to the measuring unit. The calculating unit allows to calculate a risk rate for suffering from the disease on the basis of the data acquired through the user input interface and of the at least one blood pressure value. The risk rate calculated in the calculating unit may be displayed on a display adapted therefore. The blood pressure monitor provides a risk rate for a disease automatically after each measurement.

The blood pressure values determined in the measuring unit are the systolic and the diastolic blood pressure. These are common measurement values for which statistical data in view of risks for suffering from diseases are available in the art.

The blood pressure monitor is additionally provided with a comparing unit, which allows to classify the blood pressure values in a number of predefined categories. For this purpose, the comparing unit compares, whether the blood pressure values, especially the diastolic and the systolic blood pressure each are above at least one, preferably above three different limits. Such classification allows an easy treatment of the acquired data.

At least one risk factor may be selected from the group consisting of sex and age, smoker, cholesterol level, family history and diabetes. These are typical risk factors the patients are personally aware of and which are relevant for the risk of the patient suffering from cardiovascular diseases.

According to still a further preferred embodiment of the invention, the calculating unit is designed in such a way as to calculate a limited number of different risk rates. Typically, the risk rates may be classified in no risk, low risk, medium risk, high risk and very high risk. These types of risks are easy for a patient to understand.

The blood pressure monitor according to the invention further may be provided with a memory, which is designed to permanently store the data relating to risk factors of at least one user, preferably of a plurality of users. Permanently in this context means over a longer period of time, e.g. as long as the blood pressure monitor is supplied with power. When the user for the first time uses the blood pressure monitor, he has to enter the risk factors. The risk factors are then stored in the blood pressure monitor. Each time the same user is using the blood pressure monitor thereafter reference can be made to the previously stored risk factors. Such a permanent or long term memory is, however, not necessary for the present invention. It is also conceivable to store relevant risk factors in a memory for the purpose of one measurement, whereby the data relating to the risk factors will be lost after the end of the calculating/determination procedure.

A memory for storing the risk data over a longer period of time has the advantage that the blood pressure monitor can be used by the patient without any additional interaction once these data are stored.

The risk rates are determined on the basis of the above mentioned risk factors and of the blood pressure values measured with the measuring unit.

According to a further preferred embodiment of the invention, the data are entered into said user interface as binary data. This means, that for a number of risk factors, only data such as "Yes" or "No" are entered into the interface. A total risk factor corresponding to the sum of positive risks, i.e. to the risks where the user has answered with "Yes" can be stored in the memory.

The calculation of the risk rate can therefore be easily made on the basis of the category of the blood pressure value and of the sum of positive risks stored in the memory i.e. the total risk factor. The risk factor of diabetes, however, will be treated separately as it has a stronger influence to the risk of suffering from cardiovascular disease than the other risk factors.

The blood pressure monitor may be further designed to display on said display in addition to the risk rate the blood pressure values measured with the measuring unit and/or the risk factors currently stored in the unit. The blood pressure monitor may also be designed to determine and display the pulse rate of the patient in a known way. Calculation and display of the blood pressure values and of the pulse rate are made in a manner known to those skilled in the art. Especially, the blood pressure values and the pulse rate may be determined with the oscillometric method.

The blood pressure monitor may further be provided with a communication interface, which allows to transmit the calculated risk rates to an external device such as a personal computer, a printer or a personal digital assistant.

The method according to the present invention is used to measure the blood pressure and determine at the same time a risk rate for suffering from diseases such as cardiovascular diseases. For carrying out the method according to the present invention, there is used a blood pressure monitor, especially a blood pressure monitor as described hereinbefore.

In a first step of the method, the blood pressure values are measured in an ordinary way with the blood pressure monitor. In a following step, data relating to at least one risk factor relating to the risk of suffering from the disease is read from a memory, where it is temporarily or permanently stored. On the basis of these data and of the blood pressure values, a risk rate for the specific patient for suffering from the disease is automatically calculated in the blood pressure monitor. The risk rate finally is displayed on a display. While display of the risk rate in the blood pressure monitor is preferred, other displays such as printouts or transmission to an external screen or monitor are conceivable.

After the measurement of the blood pressure values, it is determined whether the blood pressure values are above are, preferably three limits and these values are classified in a plurality of categories or grades.

For calculating the risk rate, a total risk factor consisting of the sum of a plurality of binary data may be formed. The risk rate displayed on the display may be selected from the group consisting of no risk, low risk, medium risk, high risk and very high risk.

The invention will be more clearly understood on behalf of the accompanying drawings and the following embodiment which show:
- Figure 1 -: a schematic representation of blood pressure monitor according to the invention,
- Figure 2 -: a flow chart showing the input and storage of risk factors,
- Figure 3 -: a flow chart showing the calculating operation for determining a risk rate and
- Figure 4 -: a schematic representation of the different units of the blood pressure monitor of the invention and
- Figure 5 -: an example of a display of risk rates and blood pressure values in a blood pressure monitor according to the invention.

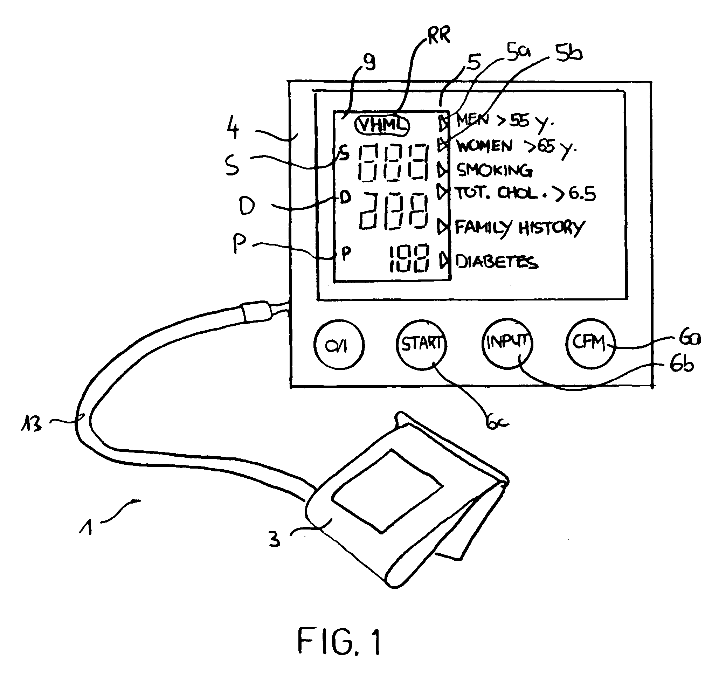

Figure 1 shows a blood pressure monitor 1 with the features according to the present invention. The blood pressure monitor 1 has a cuff 3 connected to a housing 4 of the blood pressure monitor 1 by means of a tube 13. The housing 4 is provided with a display 9. The display 9 is used to display a risk rate in a section RR, the systolic blood pressure S, the diastolic blood pressure D and the pulse rate P. In addition, the display 9 is provided with a section 5 where symbols 5a, 5b relating to risk factors may be displayed.

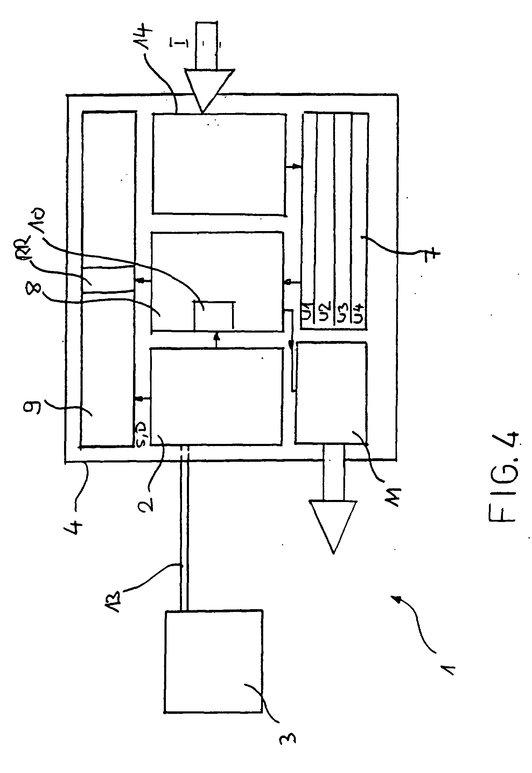

The blood pressure 1 is in addition provided with operation buttons. Mainly, there are provided a start button 6c, an input button 6b and a confirmation button 6a, which help the user to enter specific risk factors in combination with instructions appearing in the section 5 of the display 9. The buttons 6a, 6b and the symbols 5a, 5b on the display 9 together form a user interface 14 (see Figure 4) for entering risk factor data.

For starting the procedure, the user presses the input button 6b of the blood pressure monitor 1. The first of the symbols in the display section 5 represented by a triangle 5a then starts to blink. If this risk factor applies, i.e. if the user is a man with an age over 55 years, this risk factor is confirmed by pressing the CFM button 6a. The system then shifts to the next symbol represented by triangle 5b asking, whether the user is a woman with an age above 65 years.

By pressing the CFM button 6a, the respective risk factor is confirmed, i.e. the risk factor is set at "Yes". If the input button 6b is pressed, the menu will also switch to the next pointer, whereas the risk factor is set as "No". The user switches through the six symbols 5a, 5b on the display 9 indicating six different risk factors.

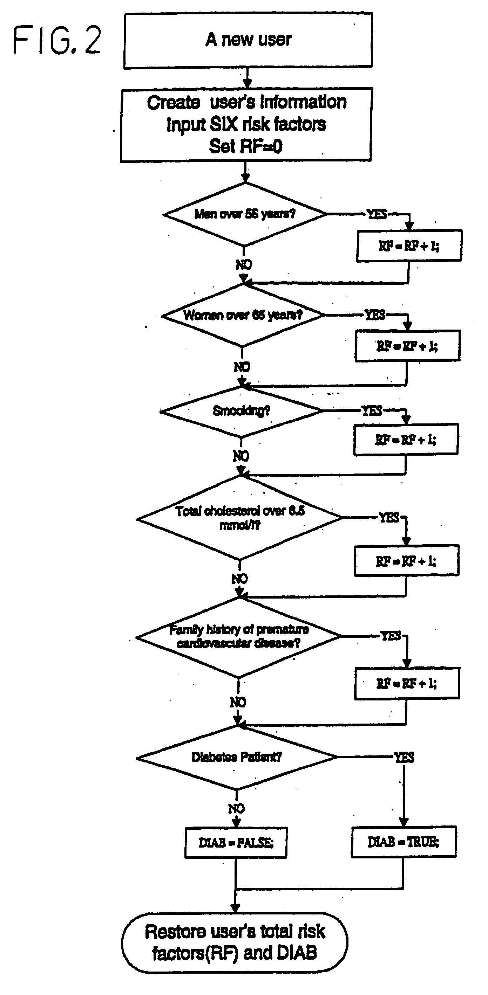

The procedure for entering the risk factors is shown in the flow chart of Figure 2. At the beginning of the set up procedure for a new user, the total risk factor RF is set to 0. Thereafter, the user is asked, whether he is a man over 55 years. If the answer is "Yes" i.e. if the CFM button is pressed, the counter for the total risk factor RF is increased by 1. If the answer is "No", the user is asked, whether he or she is a woman above 65 years. If the answer is "Yes", the counter for the total risk factor is increased by 1.

The user is then asked if he or she is a smoker. If "Yes", the counter for the total risk factor RF is increased by 1.

The user is then asked, whether his or her total cholesterol level is above 6.5 mMol per litre (250mg per dl). If the answer is "Yes", the counter for the total risk factor RF is again increased by 1.

The user is then asked, whether there is a family history of premature cardiovascular diseases. Again, if the answer is "Yes", the counter for the total risk factor RF is increased by 1.

The user is then asked, whether he is suffering from diabetes. If the answer is "Yes", a variable DIAB is set as "true". If the answer is "No", the variable DIAB is set to "false".

At the end of the procedure as shown in Figure 2, the total risk factor RF and the variable DIAB are stored in a memory 7 (see Figure 4).

The blood pressure monitor according to Figure 1 may be provided with an additional interface allowing to define and store over a longer period of time a risk factor profile for a number of users. In this case, before starting the set up procedure as shown in Figure 2, a new user must be created.

After the set up procedure for setting up risk factors, the total risk factor has a value between 0 and 4 and the variable DIAB has either a value of "false" or "true".

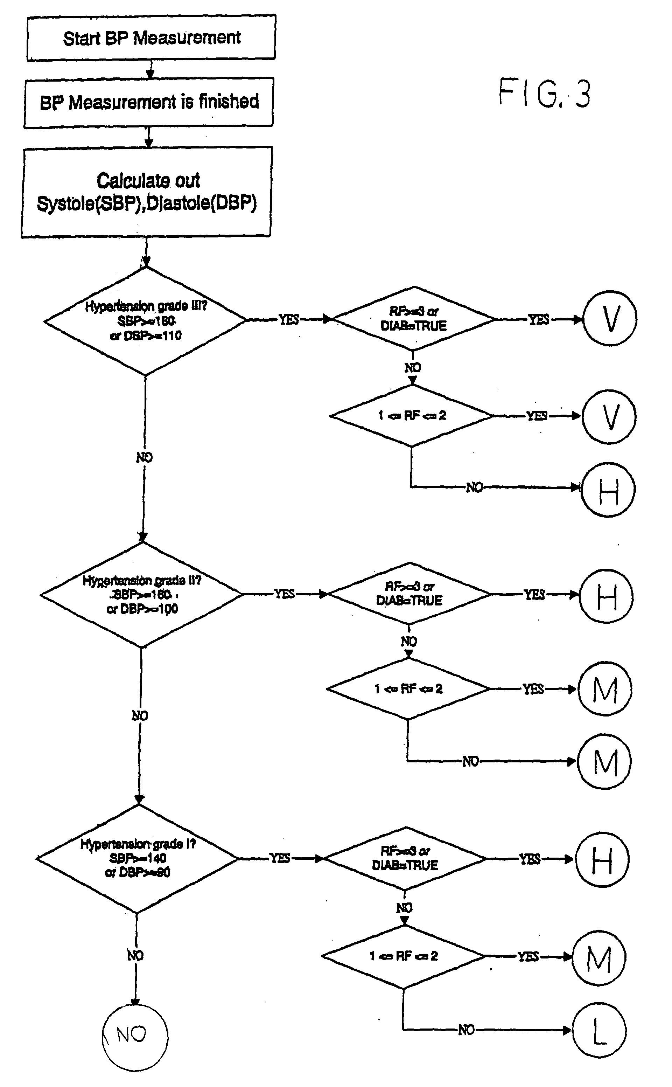

Figure 3 shows a flow chart showing the method of calculating a risk rate indicating to the patient his risk of suffering from a cardiovascular disease.

The blood pressure measurement is made in a known manner. By pushing the start button 6c, the measurement is started. At the beginning, there may be a possibility for the user to select stored risk factor profiles or to define a new risk factor profile in accordance with Figure 2. The blood pressure monitor 1 is working with the oscillometric method. During the blood pressure measurement, a pulse signal is produced. At the end of the measurement, the values of the systolic and the diastolic blood pressure are calculated on the basis of this pulse signal.

After the end of this calculation step, a classification of the blood pressure values into three categories, i.e. hypertension grade I, grade II and grade III is made. If the systolic blood pressure is above a first limit of 180 or the diastolic blood pressure is above a first limit of 110, the patient is classified as a hypertension grade III patient.

If the systolic blood pressure is above a second limit of 160 or the diastolic blood pressure is above a second limit of 100, the patient is classified as hypertension grade II.

If the systolic blood pressure is above a third limit of 140 or the diastolic blood pressure is above a third limit of 90, the patient is classified as hypertension grade I.

Calculation of a risk rate is made as follows with reference to Figure 3:

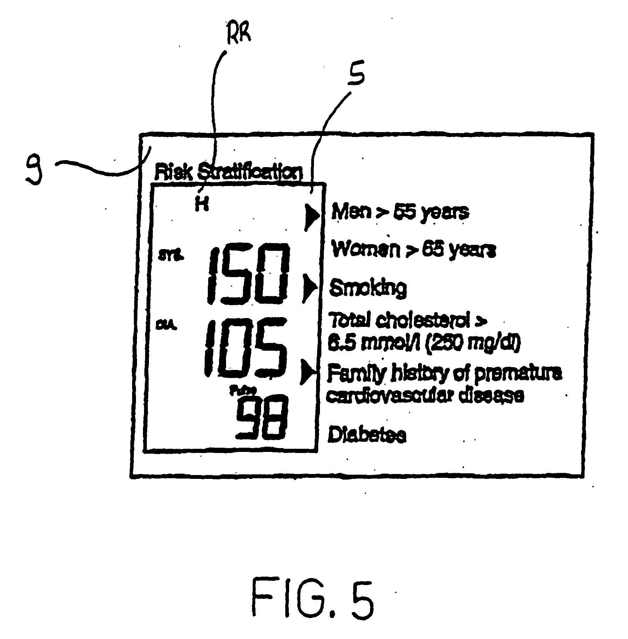

If the patient is classified as grade III, it is checked, whether the total risk factor RF is equal or greater than 3 or if the variable DIAB was previously set to "true". If "Yes", a risk rate V indicating a very high risk is displayed in the section RR of the display 9 (see Figures 1 and 5). If the check is "No", it is verified, whether the total risk factor RF is equal to 1 or 2. If the answer is "Yes", a risk rate V indicative of a very high risk is displayed. If the answer is "No", the risk rate symbol H is displayed in the section RR, which indicates high risk.

If the patient is not classified as grade III and is classified as grade II, the same tests as above is made. If the total risk factor RF is greater than or equal to 3 or if the variable DIAB is set to "true", the result of the calculation is a risk factor H indicating a high risk. If the total risk factor RF is 1 or 2, a risk rate M indicating a medium risk is displayed in the display part RR of the display 9. If the total risk factor is 0, the risk rate M is displayed.

If the patient is not classified as grade II, it is tested, whether he or she is classified as grade I. If the answer is "Yes", the same tests as described above are carried out. If the total risk factor RF is above or equal to 3, or if the variable DIAB is "true", a risk rate H is displayed. If the risk factor is 1 or 2, a risk rate M indicating a medium risk is displayed. If the risk factor is 0, a risk rate L indicating a low risk is displayed.

If the patient is not classified as grade I, i.e. if both, the systolic and the diastolic values are below the third limit, i.e. below 140 and 90 respectively, the system assumes that the risk of the patient of suffering from a cardiovascular disease is very low, so that no indication of a risk rate will be made. In this event, no symbol is displayed in the section RR of the display 9.

The blood pressure monitor according to the invention allows a stratification of risk for a specific patient suffering from a cardiovascular disease. The stratification is made according to the guidelines of 1999 of the World Health Organisation International Society of Hypertension for the management of hypertension. The system allows an easy stratification of risk rates.

An example of a possible display after determination of the blood pressure values, the pulse rate and the risk rate is shown in Figure 5.

The display 9 shows a systolic blood pressure value S of 150 and a diastolic blood pressure value D of 105 and a pulse rate P of 98. The three triangles on the display part 5 indicate, that the patient is a man with an age above 55 years, that he is a smoker and that in the family history there is an occurrence of premature cardiovascular disease. Consequently and in accordance with the flow chart of Figure 3, a risk rate H indicating a high risk is calculated and is displayed in the display part RR of the display 9.

Figure 4 shows a schematic representation of several parts of the blood pressure monitor 1 according to the present invention. The parts shown in Figure 4 are not necessarily formed as separate physical parts. They are separate operational parts, which may be implemented within one and the same microprocessor. The blood pressure monitor 1 consists of a cuff 3 connected through a tube 13 to a housing 4. Blood pressure values S, D are calculated in a measuring unit 2 to which the cuff 3 is coupled. The values of the systolic and the diastolic blood pressure S, D are then displayed in a display 9. The blood pressure monitor 1 is further provided with an interface 14 for entering input data I relating to the risk factors as shown before. The interface 14 is in fact formed by the combination of the display 5 displaying possible risk factors and the buttons 6a and 6b shown in Figure 1. Risk factors entered through the interface 14 are stored in a memory 7. The memory 7 shown in Figure 4 is designed to store risk factors for four different users U1, U2, U3 and U4. The risk factors as stored in the memory are consequently used for calculating risk rates with a calculating unit 8. The calculating unit 8 additionally includes comparing unit 10 for classifying the user into different classes of hypertension grades as shown in Figure 3. The calculating unit 8 displays risk rates as calculated in Figure 3 on the segment RR of the display 9. The blood pressure monitor 1 may further be provided with a communication interface 11 for transmitting the risk rates or also blood pressure values to an external device.

While the several units for calculating the values when carrying out the present invention are shown as separate parts, namely as a measuring unit 2, as a calculating unit 8, as a comparing unit 10, as a memory 7 and as an interface 14, these units are programmed in one and the same microprocessor. Typically, a microprocessor of the type Mitsubishi 8 bit single-chip Microprocessor 740 family/38000 series is used.

According to Figure 2, the risk factor relating to diabetes is treated differently than the other risk factors. This is in accordance with the WHO guideline for the management of hypertension. Of course, it is conceivable to weigh the risk factors differently in accordance with new scientific findings.

## Claims

1. A blood pressure monitor (1) adapted to determine a risk rate (L, H, M, V) of a patient to develop a disease, such as a cardiovascular disease, in connection with measuring the blood pressure
comprising
a measuring unit (2) adapted to measure at least the systolic (S) and the diastolic (D) blood pressure,
a user input interface (5,6a, 6b, 14) adapted to enter data of at least one predetermined risk factor relating to the risk of suffering from said disease,
a memory (7) coupled to said interface (5, 6a, 6b, 14) adapted to store said data,
a calculating unit (8) adapted to calculate a risk rate (L, M, H, V) on the basis of said data in said memory (7) and of said at least one blood pressure value (S, D) and a display (9, RR) adapted to display the risk rate (L, H, M, V) as calculated,
wherein the calculating unit (8) comprises a comparing unit (10) adapted to determine, whether the diastolic (D) or the systolic (S) blood pressure values are above at least one, preferably above three different limits and adapted to classify the patient in one of a plurality of predefined hypertension categories on the basis of the blood pressure values (D, S).

2. A blood pressure monitor according to claim 1, wherein said at least one risk factor is selected from the group consisting of sex and age, smoker, cholesterol level, family history and diabetes.

3. A blood pressure monitor according to one of the claims 1 to 2, wherein the calculating unit is designed, such as to calculate a limited number of different risk rates (L, M, H, V).

4. A blood pressure monitor according to claim 3, wherein the risk rates are no risk, low risk, medium risk, high risk and very high risk.

5. A blood pressure monitor according to one of the claims 1 to 4, wherein said memory (7) is designed to permanently store data relating to risk factors of at least one user, preferably of a plurality of users.

6. A blood pressure monitor according to one of the claims 2 to 5, wherein said data are entered into said user input interface (5, 6a, 6b,14) as binary data and wherein a total risk factor (RF) equal to the sum of positive risk factors is stored in the memory (7).

7. A blood pressure monitor according to one of the claims 1 to 6, wherein the blood pressure monitor (1) is designed to display said data relating to risk factors in a section (5) of said display (9).

8. A blood pressure monitor according to one of the claims 1 to 7, wherein the measuring unit (2) is adapted to determine the pulse rate (P) of the patient.

9. A blood pressure monitor according to one of the claims 1 to 8, wherein the blood pressure monitor is provided with a communication interface (11) for transmitting the calculated risk rates (L, M, H, V) to an external device.

## Patentansprüche

1. Blutdruckmessgerät (1) zum Bestimmen einer Risikorate (L, H, M, V) eines Patienten zur Entwicklung einer Krankheit, wie einer Herzkreislaufkrankheit, im Zusammenhang mit der Messung des Blutdrucks,
umfassend
eine Messeinheit (2) ausgebildet zum Messen wenigstens des systolischen (S) und des diastolischen (D) Blutdrucks,
eine Benutzereingabe-Schnittstelle (5, 6a, 6b, 14) ausgebildet zur Eingabe von Daten wenigstens eines vorbestimmten Risiko-Faktors betreffend das Risiko an dieser Krankheit zu erkranken,
einen Speicher (7) welcher mit der Schnittstelle (5, 6a, 6b, 14) gekoppelt ist um die Daten zu speichern,
eine Rechnereinheit (8) welche geeignet ist zum Bestimmen einer Risiko-Rate (L, M, H, V) auf der Basis der Daten in diesem Speicher (7) und des wenigstens einem Blutdruckwerts (S, D) und eine Anzeige (9, RR) welche zur Anzeige der berechneten Risikorate (L, H, M, V) ausgebildet ist,
wobei die Rechnereinheit (8) eine Vergleicheinheit (10) aufweist, welche zum Bestimmen, ob der diastolische (D) oder der systolische (S) Blutdruckwert oberhalb einem, vorzugsweise oberhalb drei verschiedenen Grenzwerten liegt, ausgebildet ist und die dafür ausgebildet ist, den Patienten in eine aus einer Vielzahl von vordefinierten Bluthochdruckkategorien auf der Basis der Blutdruckwerte (D, S) einzuteilen.

2. Blutdruckmessgerät gemäss Anspruch 1, wobei der wenigstens eine Risikofaktor ausgewählt aus der Gruppe ist, welche aus Geschlecht und Alter, Raucher, Cholesterin-Spiegel, Familiengeschichte und Diabetes besteht.

3. Blutdruckmessgerät gemäss Anspruch 1 oder 2, wobei die Rechnereinheit zum Bestimmen einer bestimmten Anzahl von verschiedenen Risikoraten (L, M, H, V) ausgebildet ist.

4. Blutdruckmessgerät gemäss Anspruch 3, wobei die Risikoraten kein Risiko, niederes Risiko, mittleres Risiko, hohes Risiko und sehr hohes Risiko sind.

5. Blutdruckmessgerät gemäss einem der Ansprüche 1 bis 4, wobei der Speicher (7) zum dauerhaften Speichern von Daten im Zusammenhang mit Risikofaktoren wenigstens eines Benutzers, vorzugsweise einer Mehrzahl von Benutzern ausgebildet ist.

6. Blutdruckmessgerät gemäss einem der Ansprüche 2 bis 5, wobei die Daten, welche in die Benutzereingabe-Schnittstelle eingegeben werden als binäre Daten eingegeben werden und wobei ein totaler Risikofaktor (RF) in dem Speicher (7) gespeichert wird, welcher der Summe der positiven Risikofaktoren entspricht.

7. Blutdruckmessgerät gemäss einem der Ansprüche 1 bis 6, wobei das Blutdruckmessgerät (1) zur Anzeige der Daten betreffend Risikofaktoren in einem Abschnitt (5) der Anzeige (9) ausgebildet ist.

8. Blutdruckmessgerät gemäss einem der Ansprüche 1 bis 7, wobei die Messeinheit (2) zur Bestimmung der Pulsrate (P) des Patienten ausgebildet ist.

9. Blutdruckmessgerät gemäss einem der Ansprüche 1 bis 8, wobei das Blutdruckmessgerät mit einer Kommunikations-Schnittstelle (11) versehen ist, um die bestimmten Risikoraten (L, M, H, V) zu einem externen Gerät zu übermitteln.

## Revendications

1. Appareil de mesure de la pression sanguine (1), adapté pour déterminer un taux de risque (L, H, M, V) de contraction, par un patient, d'une maladie, comme une maladie cardiovasculaire, en combinaison avec la mesure de la pression sanguine
comprenant
une unité de mesure (2) adaptée pour mesurer au moins la pression sanguine systolique (S) et diastolique (D),
une interface-utilisateur de saisie (5, 6a, 6b, 14) adaptée pour saisir les données d'au moins un facteur de risque prédéterminé concernant le risque d'être atteint de ladite maladie,
une mémoire (7) couplée à ladite interface (5, 6a, 6b, 14) adaptée pour enregistrer lesdites données, une unité de calcul (8) adaptée pour calculer un taux de risque (L, M, H, V) en fonction desdites données dans ladite mémoire (7) et de ladite au moins une valeur de pression sanguine (S, D), et
un affichage (9, RR) adapté pour afficher le taux de risque (L, H, M, V) ainsi calculé,
dans lequel l'unité de calcul (8) comprend une unité de comparaison (10) adaptée pour déterminer si les valeurs de pression sanguine diastolique (D) ou systolique (S) sont supérieures à au moins une, de préférence supérieur à trois limites, et adapté pour classer le patient dans une pluralité de catégories d'hypertension prédéterminées en fonction des valeurs de pression sanguine (D, S).

2. Appareil de mesure de la pression sanguine selon la revendication 1, dans lequel ledit au moins un facteur de risque est choisi dans le groupe comprenant le sexe et l'âge, le tabagisme, le niveau de cholestérol, les antécédents familiaux et le diabète.

3. Appareil de mesure de la pression sanguine selon l'une des revendications 1 et 2, dans lequel l'unité de calcul est conçue de façon à calculer un nombre limité de taux de risque différents (L, M, H, V).

4. Appareil de mesure de la pression sanguine selon la revendication 3, dans lequel les taux de risque sont : aucun risque, faible risque, risque moyen, risque élevé et risque très élevé.

5. Appareil de mesure de la pression sanguine selon l'une des revendications 1 à 4, dans lequel ladite mémoire (7) est conçue pour enregistrer de façon permanente des données concernant les facteurs de risque d'au moins un utilisateur, de préférence d'une pluralité d'utilisateurs.

6. Appareil de mesure de la pression sanguine selon l'une des revendications 2 à 5, dans lequel lesdites données sont entrées dans ladite interface-utilisateur de saisie (5, 6a, 6b, 14) sous forme de données binaires, et dans lequel un facteur de risque total (RF) égal à la somme de facteurs de risque positifs est enregistré dans la mémoire (7).

7. Appareil de mesure de la pression sanguine selon l'une des revendications 1 à 6, dans lequel l'appareil de mesure de la pression sanguine (1) est conçu pour afficher lesdites données concernant des facteurs de risque dans une section (5) dudit affichage (9).

8. Appareil de mesure de la pression sanguine selon l'une des revendications 1 à 7, dans lequel l'unité de mesure (2) est adaptée pour déterminer le pouls (P) du patient.

9. Appareil de mesure de la pression sanguine selon l'une des revendications 1 à 8, dans lequel l'appareil de mesure de la pression sanguine est pourvu d'une interface de communication (11) pour transmettre les taux de risque calculés (L, M, H, V) à un dispositif externe.
